(19) Europäisches Patentamt
European Patent Office
Office européen des brevets

(11) EP 1 510 511 A1

(12) **EUROPEAN PATENT APPLICATION**
published in accordance with Art. 158(3) EPC

(43) Date of publication:
**02.03.2005 Bulletin 2005/09**

(21) Application number: **03730610.7**

(22) Date of filing: **23.05.2003**

(51) Int Cl.[7]: **C07C 43/178**, C07C 43/23, C07C 49/255, A61K 7/00, A61K 7/48, A61K 31/075, A61K 31/085, A61P 31/12, A61P 17/00, A61P 39/06, A61P 43/00

(86) International application number:
**PCT/JP2003/006470**

(87) International publication number:
**WO 2003/099752 (04.12.2003 Gazette 2003/49)**

(84) Designated Contracting States:
**AT BE BG CH CY CZ DE DK EE ES FI FR GB GR HU IE IT LI LU MC NL PT RO SE SI SK TR**

(30) Priority: **27.05.2002 JP 2002152725**
**22.01.2003 JP 2003014030**

(71) Applicant: **TOAGOSEI CO., LTD.**
**Tokyo 105-8419 (JP)**

(72) Inventors:
- **KATO, Hisatoyo, Toagosei Co., Ltd.**
  **Tsukuba-shi, Ibaraki 300-2611 (JP)**
- **YAMAGUCHI, Shuhei, Toagosei Co., Ltd.**
  **Tsukuba-shi, Ibaraki 300-2611 (JP)**

(74) Representative: **Pohlmann, Eckart, Dipl.-Phys.**
**WILHELMS, KILIAN & PARTNER,**
**Patentanwälte,**
**Eduard-Schmid-Strasse 2**
**81541 München (DE)**

(54) **GINGEROL ANALOGUES AND USE THEREOF**

(57) The invention provides novel compounds with a chemical structure similar to that of gingerol useful as a component for foods, pharmaceutical products, cosmetics and the like.

After intensive investigations, the inventors found the gingerol analogues represented by the following general formulae (1) and (2). Additionally, the inventors found that these compounds had excellent properties as an inhibitor of tyrosinase activity and an antioxidant.

By using the following compounds of the invention as a component for cosmetics, for example, cosmetics suppressing the generation of speckles and spots can be produced. By using the compounds as a component for foods, quality deterioration due to the oxidation of foods can be prevented.

Gingerol analogues represented by the following general formula (1).

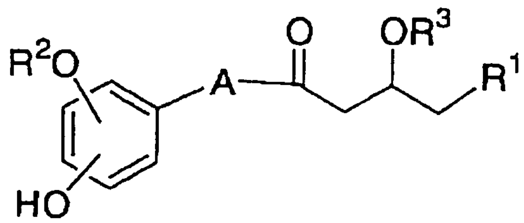

(1)

(In the formula (1) , $R^1$ represents an alkyl group with one to 18 carbon atoms; $R^2$ is hydrogen atom or methyl group; $R^3$ is methyl group or ethyl group; and A represents an alkylene group with one to 4 carbon atoms.)

Gingerol analogues represented by the following general formula (2).

EP 1 510 511 A1

Printed by Jouve, 75001 PARIS (FR)

(Cont. next page)

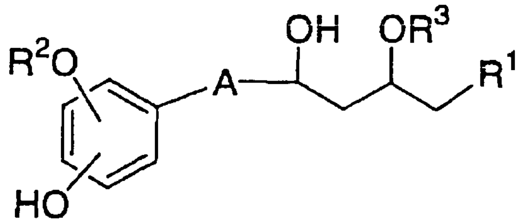

(2)

(In the formula (2), $R^1$ represents an alkyl group with one to 18 carbon atoms; $R^2$ is hydrogen atom or methyl group; $R^3$ is methyl group or ethyl group; and A represents an alkylene group with one to 4 carbon atoms.)

## Description

Technical Field

The present invention relates to novel compounds with a chemical structure similar to naturally occurring gingerol useful in the fields of foods, pharmaceutical products, qui-pharmaceutical products, cosmetics and the like (referred to as gingerol analogues hereinafter), as well as the use thereof. The gingerol analogues of the invention have excellent properties as an inhibitor of tyrosinase activity and an antioxidant and can be added to the foods, pharmaceutical products or cosmetics or the like.

Background Art

Speckles and spots emerge due to abnormal deposition of melanin dye formed in pigment cells of human epidermis because of ultraviolet exposure and the like. Melanin dye is synthesized by metabolic modification of L-tyrosine as one of amino acids into L-dopa and L-dopaquinone with tyrosinase as an oxidative enzyme and subsequent various pathways (references: Journal of Fine Chemicals, No. March 15, 1999 issue, Special Edition, Development and Products of Whitening Agents; and Journal of Fragrance, No. September 1997 issue, Special Edition, Recent Progress of Researches and Development of Whitening Agent). Thus, inhibition of tyrosinase activity is effective for preventing speckles and spots.

Meanwhile, it has been elucidated recently that active oxygen causes various diseases. Active oxygen is generated in the biological immune system and plays a role in attacking pathological bacteria invaded from outside. In case that it is generated excessively, it reacts with lipids, proteins and DNA and the like composing biological organisms, disadvantageously leading to adverse influences on biological organisms. Unsaturated fatty acids composing biomembrane and the like are particularly readily oxidized, so that the fatty acids generate lipid peroxide via oxidation. The resulting lipid peroxide is known to induce arteriosclerosis, hypertension and liver malfunction. Additionally when unsaturated fatty acids generally contained in foods, pharmaceutical products, cosmetics and the like are oxidized to generate lipid peroxide, the quality of the products described above is deteriorated.

As a measure for solving the problem, the use of antioxidants is listed. The antioxidants for use in the fields of foods, pharmaceutical products, qui-pharmaceutical products, cosmetics and the like are preferably naturally occurring antioxidants with less toxicity to humans. Specific naturally occurring antioxidants include for example tocopherols, ascorbic acid and derivatives thereof, ubiquinone and derivatives thereof, flavone derivatives, gallate derivatives, and polyphenols (Kenji Fukuzawa, "Pharmacology of Free Radical Protection and Development of Pharmaceutical Drugs therefor", Nippon Rinsho (Japanese Journal of Clinical Medicine), October 1988, Vol.46, No.10, p. 2269-2276, and Sies, H., and two others, "Antioxidant Functions of Vitamins", Annals of New York Academy of Sciences, 1992, Vol. 669, p. 7-20).

It is an object of the invention to provide a compound functioning as an inhibitor of tyrosinase activity or an antioxidant, which is to be added to products for example foods, pharmaceutical products, qui-pharmaceutical products, cosmetics and the like.

Disclosure of the Invention

The present inventors made investigations so as to solve the problem. Consequently, the inventors found that a compound similar to gingerol had an inhibitory property of tyrosinase activity and an antioxidant action. Thus, the invention has been achieved.

A first aspect of the invention is gingerol analogues represented by the following general formula (1):

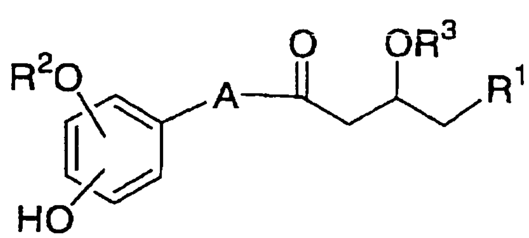

(1)

(in the formula (1), $R^1$ represents an alkyl group with one to 18 carbon atoms; $R^2$ is hydrogen atom or methyl group;

$R^3$ is methyl group or ethyl group; and A represents an alkylene group with one to 4 carbon atoms.)

A second aspect of the invention is gingerol analogues represented by the following general formula (2):

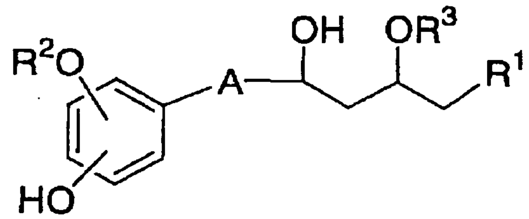

(2)

(in the formula (2), $R^1$ represents an alkyl group with one to 18 carbon atoms; $R^2$ is hydrogen atom or methyl group; $R^3$ is methyl group or ethyl group; and A represents an alkylene group with one to 4 carbon atoms.)

A third aspect of the invention is an inhibitor of tyrosinase activity, which includes gingerol analogues represented by the general formula (1) or (2).

A fourth aspect of the invention is an antioxidant, which includes gingerol analogues represented by the general formula (1) or (2).

The invention is now described in more detail hereinbelow. In the general formula (1) representing gingerol analogues in the first aspect of the invention, $R^1$ represents an alkyl group with one to 18 carbon atoms, which may or may not have branched chains, and includes for example methyl group, ethyl group, propyl group, butyl group, isobutyl group, pentyl group, hexyl group, peptyl group, octyl group, 2-ethylhexyl group, nonyl group, decyl group, lauryl group, and stearyl group. Preferably, $R^1$ is a linear alkyl group. Taking account of the structure of gingerol existing in the natural kingdom, $R^1$ preferably has carbon atoms of an even number and particularly preferably includes ethyl group, butyl group, hexyl group, octyl group and decyl group.

In the formula (1), $R^2$ is hydrogen atom or methyl group, preferably methyl group.

In the formula (1), $R^3$ is methyl group or ethyl group, preferably methyl group.

In the formula (1), A is an alkylene group with one to 4 carbon atoms, preferably ethylene group or butylene group, more preferably ethylene group.

One example of the gingerol analogues represented by the general formula (1) can be prepared readily for example by chemical synthesis or by adding methanol or ethanol to a compound represented by the general formula (3), which is from natural origins.

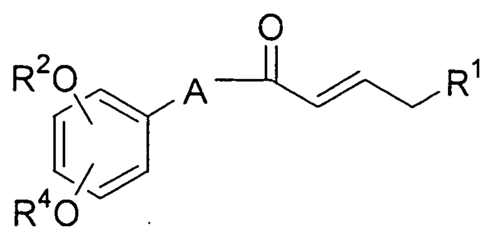

(3)

In the formula (3), $R^1$ corresponds to $R^1$ in the formula (1). $R^2$ and $R^4$ each independently is hydrogen atom, a lower alkyl group or a protective group of phenolic hydroxyl group. Herein, at least one of $R^2$ and $R^4$ should be hydrogen atom or a protective group of phenolic hydroxyl group.

When $R^2$ and/or $R^4$ is a protective group of phenolic hydroxyl group in the general formula (3), the protective group is preferably silyl-type protective groups and acyl-type protective groups, taking account of simple introduction of the protective group and simple elimination thereof. Specifically, the protective group includes for example trimethylsilyl group, t-butyldimethylsilyl group, acetyl group, propionyl group, butyroyl group, isobutyroyl group, pivaloyl group, benzoyl group, and toluoyl group. More preferably, the protective group is acetyl group, propionyl group, butyroyl group, isobutyroyl group, pivaloyl group, benzoyl group and toluoyl group.

The addition reaction of methanol or ethanol to a compound represented by the general formula (3) is preferably done in the presence of an alkali catalyst. The alkali catalyst includes for example sodium hydroxide, potassium hydroxide, metal alkoxides such as sodium methoxide or sodium ethoxide, and sodium hydride. The amount of such catalyst to be used varies depending on the structures of $R^2$ and $R^4$ in the general formula (3) and the type of the catalyst. The amount is 0.05 to 10 chemical equivalents, preferably 0.1 to 5 chemical equivalents. When the amount of the catalyst used is too less, the reaction progresses slowly. When the amount is too much, a greater amount of a

neutralization agent is needed after the reaction.

The addition reaction may satisfactorily be done in a solvent. As the solvent, preferably, tetrahydrofuran, 1,4-dioxane, diethyl ether, 1,2-dimethoxyethane, N,N-dimethylformamide, hexamethylphosphoric triamide, N,N-dimethylpropylene urea, isopropyl alcohol, water and mixture solvents thereof are used.

The reaction temperature is preferably -20 °C to 100 °C, more preferably 0 °C to 80 °C. When the reaction temperature is too low, the reaction progresses slowly. When the reaction temperature is too high, side reactions proceed. The reaction time is appropriately several tens of minutes to several hours.

After completion of the reaction, the gingerol analogues represented by the general formula (1) can be obtained by known processes such as solvent extraction and column chromatography.

The compound represented by the general formula (3) [referred to as compound of the formula (3) hereinbelow] can be synthetically prepared by synthetically preparing a compound represented by the following formula (6) [referred to as compound of the formula (6) hereinbelow] from a compound represented by the following formula (4) [referred to as compound of the formula (4) hereinbelow] and a compound represented by the following formula (5) [referred to as compound of the formula (5) hereinbelow] as raw materials and then eliminating HX from the resulting compound of the formula (6).

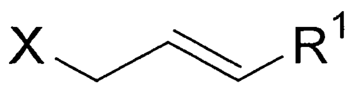 (4)

In the formula (4), $R^1$ is an alkyl group with one to 18 carbon atoms and X is benzenesulfonyl group or toluenesulfonyl group.

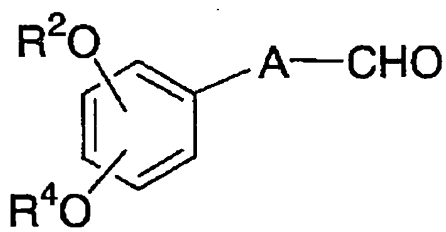 (5)

In the formula (5), $R^2$ and $R^4$ correspond to $R^2$ and $R^4$ in the formula (3) respectively and A is an alkylene group with one to 4 carbon atoms.

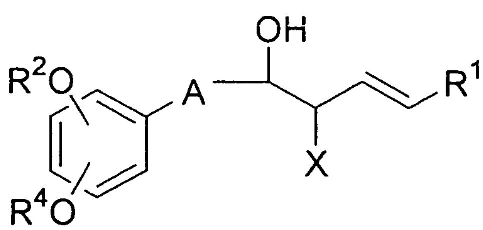 (6)

In the formula (6), $R^2$ and $R^4$ correspond to $R^2$ and $R^4$ in the formula (3) respectively and A is an alkylene group with one to 4 carbon atoms. $R^1$ is an alkyl group with one to 18 carbon atoms, and X is benzenesulfonyl group or toluenesulfonyl group.

The compound of the formula (4) can be synthetically prepared by synthetic methods described in for example a reference by K. Inomata, et al., Chem. Lett., 931 (1985).

Additionally, the compound of the formula (5) can be synthetically prepared by synthetic methods described in for example a reference by G. Solladie, et al., J. Org. Chem., 58, 2181 (1993).

A in the formula (5) is preferably ethylene group or butylene group, more preferably ethylene group.

The compound of the formula (6) can be synthetically prepared by reacting an alkyl metal compound with the compound of the formula (4) and further reacting the compound of the formula (5) with the resulting product. In other words, an alkyl metal compound attacks the compound of the formula (4) to withdraw hydrogen atom from carbon bound with a substituent -X, to thereby generate carbon anion based on the compound of the formula (4). When the

compound of the formula (5) is subsequently added to the reaction system, the carbon anion reacts with the aldehyde group in the compound of the formula (4) to synthetically prepare the formula (6).

The alkyl metal compound for use in the reaction for withdrawing hydrogen atom from the compound of the formula (4) includes for example alkyl lithium compounds such as n-butyllithium, s-butyllithium, t-butyllithium and phenyllithium; and Grignard reagents such as n-butylmagnesium chloride, s-butylmagnesium chloride, t-butylmagnesium chloride, n-butylmagnesium bromide, s-butylmagnesium bromide, and t-butylmagnesium bromide. Preferably, the alkyl metal compound is n-butyllithium, n-butylmagnesium chloride or n-butylmagnesium bromide.

Instead of the alkyl metal compound, additionally, alkali metals such as metal lithium and metal sodium may satisfactorily be used.

The amount of such alkyl metal compound to be used is preferably 0.7 to 1.3 chemical equivalents, more preferably 0.9 to 1.1 chemical equivalents to that of the compound of the formula (4).

The reaction between the compound of the formula (4) and the alkyl metal compound is preferably carried out in a aprotic solvent. As such, tetrahydrofuran, 1,4-dioxane, diethyl ether, 1,2-dimethoxyethane, hexamethylphosphoric triamide, N,N-dimethylpropylene urea, and mixture solvents thereof can preferably be used.

The reaction temperature for the reaction between the compound of the formula (4) and the alkyl metal compound is preferably -100 °C to 25 °C, more preferably -80 °C to 0 °C. When the reaction temperature is too low, the temperature is retained at high cost. When the reaction temperature is too high, side reactions proceed. The reaction time is appropriately several minutes to several tens of minutes.

Then, the compound of the formula (5) is added to the reaction solution obtained by the reaction of the compound of the formula (4) with the alkyl metal compound. The temperature of the reaction solution then is preferably -100 °C to 25 °C. The reaction time is generally several minutes to several tens of minutes.

After completion of the reaction, the compound of the formula (6) can be isolated and purified by known methods such as solvent extraction and column chromatography.

As described above, the compound of the formula (3) can be synthetically prepared by eliminating HX from the compound of the formula (6). In other words, π-allyl complex is formed by reacting the ethylenic unsaturated bond in the compound of the formula (6) with a metal catalyst. Then, the compound of the formula (3) can be prepared synthetically by reacting the resulting π-allyl complex with a basic compound.

As such metal catalyst, metal catalysts capable of forming π-allyl complexes together with the ethylenic unsaturated bond in the compound of the formula (6) can be used. Palladium complex can preferably be used. Specifically, the metal catalyst includes for example tetrakis-triphenylphosphine palladium (valence zero), tris(dibenzylideneacetone) dipalladium (valence zero), chloroform adducts, palladium chloride (divalence)/triphenylphosphine mixture, palladium acetate (divalence)/triphenylphosphine mixture, and palladium acetate (divalence)/tributylphosphine mixture. The description in parentheses means the number of the valence of metal palladium in each of the compounds.

The amount of such metal catalyst to be used is preferably 0.0001 to 1 mol, more preferably 0.001 to 0.1 mol per one mol of the compound of the formula (6).

As such basic compound, tertiary amines such as triethylamine, diisopropylethylamine, N-methylimidazole and pyridine are preferable. The amount of such basic compound to be used is 0.9 mole or more per one mole of the compound of the formula (6), and is preferably within a range of 1.0 mole to 10 moles. Additionally, such basic compound may satisfactorily be used as the solvent.

The reaction is preferably carried out in the presence of a solvent. As such solvent, tetrahydrofuran, 1,4-dioxane, diethyl ether, 1,2-dimethoxyethane, acetonitrile, chloroform, dichloromethane, 1,2-dichloroethane, hexamethylphosphoric triamide, N,N-dimethylpropylene urea, methanol, ethanol, isopropyl alcohol, ethylene glycol, glycerin and mixture solvents thereof can be used. Among them, a mixture of 1,2-dichloroethane and alcohol is preferable.

The reaction temperature is preferably ambient temperature to 150 °C, more preferably 50 °C to 120 °C. The reaction time is appropriately several hours to several tens of hours.

After completion of the reaction, the compound of the general formula (3) can be obtained by known methods such as solvent extraction and column chromatography.

The gingediol analogues represented by the general formula (2) is now described.

The gingediol analogues represented by the general formula (2) can be synthetically prepared for example by reduction of the carbonyl group in the compound represented by the general formula (1).

As such reducing reagent for use in the reduction reaction, metal hydrides such as lithium aluminium hydride, lithium borohydride, and sodium borohydride can be used preferably with no specific limitation. The preferable amount of metal hydrides when used varies depending on the type of a metal hydride and the structure of $R^2$ in the general formula (1). In case that $R^2$ in the general formula (1) is methyl group and lithium aluminum hydride is used as such reducing reagent, 0.5 to 3 moles of lithium aluminium hydride are preferable per one mole of the compound represented by the general formula (1).

For the reaction, solvents may satisfactorily be used. As such solvent, tetrahydrofuran, 1,4-dioxane, diethyl ether, 1,2-dimethoxyethane, diglyme and mixture solvents thereof can preferably be used.

In case that metal hydrides are used as reducing reagents, the reaction temperature is preferably -30 °C to 100 °C, while the reaction time is generally several minutes to several tens of minutes. After completion of the reaction, the intended compound can be obtained by known methods such as solvent extraction and column chromatography.

As shown in experimental results below in Examples, it was confirmed that the compound represented by the general formula (1) and the compound represented by the general formula (2) had a property of inhibiting the activity of tyrosinase as an oxidative enzyme of L-tyrosine. Further, it was anticipated that because all the compounds described above had phenolic hydroxyl group, the compounds had a property as an antioxidant. It was confirmed at experiments that the property was sufficiently great.

Specific uses of these compounds include for example blending the compounds in foods, pharmaceutical products, qui-pharmaceutical products, cosmetics such as cosmetic lotion, aesthetic lotion, emulsion, cream and pack, and the like.

Brief Description of the Drawing

Fig.1 shows test results of the inhibition of tyrosinase activity using L-dopa as substrate, as carried out in Example 5 described below, where A1, A2, B1 and B2 individually express the following samples; and the unit of numerical figures on the longitudinal axis is %. The numerical figures show the dimension of the inhibition ratio of tyrosinase activity.

A1 and A2: samples prepared by adding 0.27 μmol (A1) or 0.54 μmol (A2) of the compound of the formula (11) as obtained in Example 1 to 3 ml of an enzyme reaction solution.
B1 and B2: samples prepared by adding 0.27 μmol (B1) or 0.54 μmol (B2) of albumin as a standard compound to 3 ml of an enzyme reaction solution.

Best Mode for Carrying out the Invention

The invention is specifically described in the following Synthetic Examples, Examples and Comparative Examples. In the following individual formulas, herein, Ts represents p-toluenesulfonyl group.

Synthetic Example 1

The compound of the formula (9) was synthetically prepared from the compound of the formula (7) and the compound of the formula (8).
Compound of the formula (7);

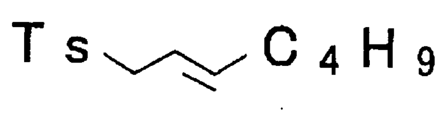

(7)

Compound of the formula (8);

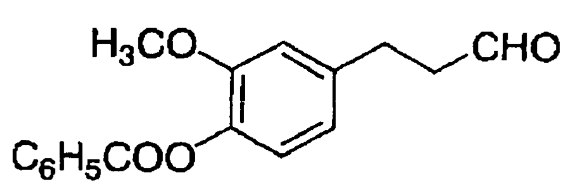

(8)

Compound of the formula (9);

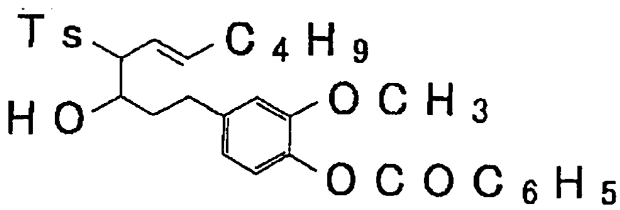

(9)

Further, Ts was removed from the compound of the formula (9) in the presence of a palladium catalyst, to synthetically prepare the compound represented by the following formula (10), namely [6]-shogaol.
[6]-Shogaol:

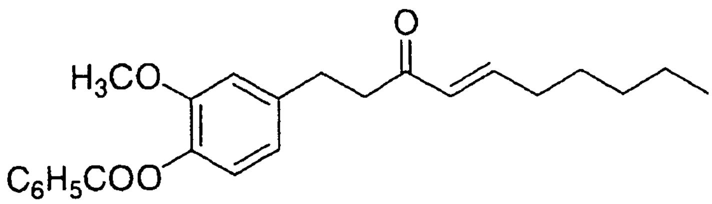

(10)

3.46 g (13.7 mmol) of the compound of the formula (7) was dissolved in 50 ml of tetrahydrofuran, and the resulting solution was cooled to -78 °C with dry ice/acetone. To the resulting solution was dropwise added 9.10 ml (13.7 mmol) of 1.50 M n-butyllithium/n-hexane solution. After the resulting mixture was agitated at the same temperature for 20 minutes, a solution of 3.49 g (13.0 mmol) of the compound of the formula (8) as dissolved in 50 ml of tetrahydrofuran was dropwise added to the mixture. After dropwise addition, the resulting mixture was agitated at the same temperature for 10 minutes. Then, the temperature was gradually elevated. When the temperature of the reaction solution reached -10 °C, 2 ml of methanol was added to terminate the reaction. After 30 ml of saturated aqueous sodium chloride was added to the reaction mixture, the mixture was agitated, to separate the organic layer. The aqueous layer was extracted in 30 ml of ethyl acetate. The resulting organic layers were combined together and dried over anhydrous magnesium sulfate. After the solvent was distilled off, purification by silica gel column chromatography was done to obtain a highly viscous, liquid compound of 5.79 g in pale yellow (yield of 79 %).

The chemical shift values of the product on $^1$H-NMR spectrum as measured in chloroform-d1 were as follows: 0.72-0.89(3H, m), 0.98-1.24(4H, m), 1.52-2.04(4H, m), 2.45(3H, s), 2.82-2. 94 (2H, m), 3.16-3.64(1H, m), 3.79 (3H, s), 4.04-4.65(2H, m), 5.03-5.85(2H, m), 6.70-6.84(2H, m), 6.96-7.05(1H, m), 7.32(2H, d), 7.45-7.75(5H, m), 8.20(2H, d).

Additionally, the wave numbers ($cm^{-1}$) with absorption by IR absorption spectrometry (KBr pellet method) were as follows: 3520, 2950, 2930, 2870, 1740, 1600, 1510, 1450, 1280, 1260, 1200, 1140, 1120, 1080, 1060, 1020, 710.

Further, the results of elemental analysis were as follows: 69.22 % of carbon and 6.55 % of hydrogen. Based on the analysis, it was verified that the obtained compound was the compound of the formula (9).

1.31 g (2.51 mmol) of the compound of the formula (9) was dissolved in a mixture solvent of 39 g of 1,2-dichloroethane, 13 g of isopropyl alcohol and 13 g of glycerin. To the resulting solution were added 1.05 ml (7.53 mmol) of triethylamine, 39.5 mg (0.151 mmol) of triphenylphosphine, and 87.0 mg (0.0753 mmol) of tetrakistriphenylphosphine palladium, and the resulting mixture was agitated at a bath temperature of 100 °C for 18 hours. After the solvent and the like were distilled off, then, 50 ml of distilled water, 20 ml of aqueous saturated sodium chloride and 50 ml of ethyl acetate were added to the resulting product, for partition. The resulting organic layer was separated and isolated. The aqueous layer after extraction was again extracted in 20 ml of ethyl acetate. The combined organic layer was dried over anhydrous magnesium sulfate and then concentrated. The resulting reaction mixture was purified by silica gel column chromatography, and recrystallized in a mixture solvent of ethyl acetate and n-hexane, to obtain a compound of 603 mg in colorless crystal (yield of 63 %).

The chemical shift values of the product on $^1$H-NMR spectrum as measured in chloroform-d1 were as follows: 0.91(3H, t), 1.25-1.60(6H, m), 2.21(2H, q), 2.85-3.00(4H, m), 3.82(3H, s), 6.16(1H, d), 6.79-6.94(3H, m), 7.05(1H, d), 7.45-7.68(3H, m), 8.20(2H, d).

Additionally, the wave numbers ($cm^{-1}$) with absorption by IR absorption spectrometry (KBr pellet method) were as follows: 2950, 2930, 2870, 1730, 1660, 1600 1510, 1470, 1450, 1420, 1270, 1200, 1150, 1060, 710.

Further, the results of CHN elemental analysis were as follows: 75.56 % of carbon and 7.70 % of hydrogen.

Based on the analysis, it was verified that the obtained compound was the compound of the formula (10).

Example 1

Methanol was added to the compound represented by the formula (10) as obtained in the Synthetic Example 1 in the presence of sodium hydroxide, while benzoyl group was removed from the compound, to obtain the compound of the following formula (11) as one example of the gingerol analogues of the invention.
Compound of the formula (11);

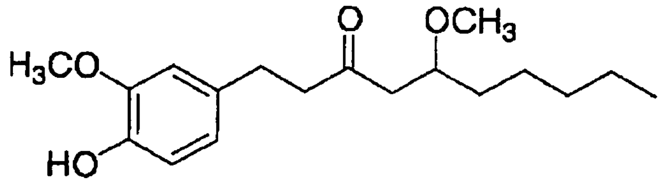

(11)

330 mg (0.867 mmol) of the compound represented by the formula (10) was dissolved in 2 ml of isopropyl alcohol, followed by addition of 8 ml of methanol and 0.9 ml of aqueous 1N sodium hydroxide solution, for agitation at ambient temperature for 2 hours. Subsequently, 2 ml of 0.5N hydrochloric acid was added to terminate the reaction. After methanol in the reaction solution was distilled off, 25 ml of aqueous saturated sodium chloride was added, for extraction twice in 25 ml of ethyl acetate. These ethyl acetate were combined together, dried over anhydrous magnesium sulfate and concentrated. The resulting concentrate was purified by silica gel thin layer chromatography, to obtain a colorless compound in liquid at 191 mg (71 %).
The chemical shift values of the product on $^1$H-NMR spectrum as measured in chloroform-d1 were as follows: 0.89(3H, t), 1.20-1.83(8H, m), 2.34-2.89(6H, m), 3.28(3H, s), 3.57-3.70(1H, m), 3.86(3H, s), 5.49(1H, s), 6.63-6.73 (2H, m) , 6.81 (1H, d).
Additionally, the wave numbers ($cm^{-1}$) with absorption by IR absorption spectrometry (KBr pellet method) were as follows: 3400, 2930, 2860, 1710, 1600, 1510, 1460, 1450, 1430, 1370, 1270, 1230, 1090, 1030.
Further, the results of elemental analysis were as follows: 69.80 % of carbon and 8.98 % of hydrogen. Based on the analysis, it was verified that the obtained compound was the compound of the formula (11).

Example 2

The compound of the formula (11) as obtained in Example 1 was reduced, to synthetically prepare an additional type of the gingerol analogues as represented by the following formula (12).
Compound of the formula (12);

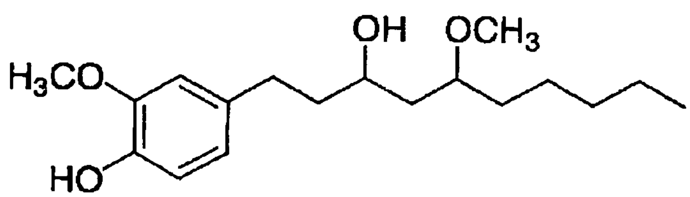

(12)

A solution of 154 mg (0.50 mmol) of the compound of the formula (11) in 5 ml of tetrahydrofuran was dropwise added to a solution of 19.0 mg (0.50 mmol) of lithium aluminium hydride in 1 ml of tetrahydrofuran, for agitation at ambient temperature for 16 hours. Subsequently, 5 ml of distilled water and 2 ml of 0.5N hydrochloric acid were dropwise added, for extraction three times in 10 ml of ethyl acetate. The organic layer was dried over anhydrous magnesium sulfate and purified by silica gel thin layer chromatography, to obtain a colorless compound in liquid at 146 mg (94 %).
The chemical shift values of the product on $^1$H-NMR spectrum as measured in chloroform-d1 were as follows: 0.89(3H, t), 1.20-1.87(12H, m), 2.52-2.81(2H, m), 3.30-3.55(4H, m), 3.72-4.00(4H, m), 5.55(1H, s), 6.65-6.75(2H, m), 6.81(1H, d). Additionally, the wave numbers ($cm^{-1}$) with absorption by IR absorption spectrometry (KBr pellet method) were as follows: 3390, 2930, 2860, 1600, 1510, 1460, 1450, 1430, 1370, 1270, 1230, 1080, 1030.
Further, the results of elemental analysis were as follows: 69.74 % of carbon and 9.47 % of hydrogen. Based on the analysis, it was verified that the obtained compound was the compound of the formula (12).

Example 3

(Test about suppression of lipid peroxide generation)

Using the compound of the formula (11) as obtained in Example 1 and the compound of the formula (12) as obtained in Example 2, a test about the suppression of lipid peroxide generation in linoleic acid was done. Along with the compounds, dl-α-tocopherol (manufactured by Tokyo Kasei Kogyo Co., Ltd.) was tested similarly, to obtain control data.

The test procedures are as follows. As a test result, the ratio of residual linoleic acid by the following procedure (3) is described in Table 1.

(1) After ethanol (2.0 g ) was added to linoleic acid (0.2 g; manufactured by Tokyo Kasei Kogyo Co., Ltd.), a test compound (0.01 g) and a surface active agent Nissan OT-221 (0.4 g; manufactured by NOF Corporation) placed in a 50-ml screw vial, for dissolution, distilled water (17.39 g) was added to the resulting solution for agitation, and was then sealed and left to stand alone in a thermostat at 40 °C. Such sample was prepared in a duplex manner.

A blank test with no test compound added was similarly done.

(2) The amount of remaining linoleic acid one week, 2 weeks, 3 weeks and 4 weeks after the start of the test was assayed by HPLC.

The HPLC analytical conditions were as follows. Wavelength for detection: 210 nm. Mobile phase: a mixture solution of a disodium hydrogen phosphate-phosphate buffer adjusted to pH 2.5 and methanol (10:90). Flow: 1 ml/min. Column: ODS-80Ts (4.6 Φmm × 150 mm). Column temperature at 40 °C.

(3) Each sample was assayed by HPLC two times. The ratio of remaining linoleic acid was calculated on the basis of the mean of four assay values. The results are shown in Table 1.

Table 1

| | | | | (unit: %) |
|---|---|---|---|---|
| | One week later | 2 weeks later | 3 weeks later | 4 weeks later |
| Blank | 73.1 | 53.6 | 38.0 | 23.4 |
| Compound of formula (11) | 90.5 | 86.1 | 80.5 | 80.9 |
| Compound of formula (12) | 89.2 | 85.5 | 82.7 | 80.9 |
| Tocopherol | 83.6 | 73.2 | 62.6 | 57.3 |

Example 4

(Test about scavenging DPPH radical)

Concerning radical-scavenging activity, a stable radical DPPH (1,1-diphenyl-2-picrylhydrazyl) was used for assaying the radical-scavenging activities of test compounds [the compound of the formula (11) and the compound of the formula (12) and a control sample dl-α-tocopherol (manufactured by Tokyo Kasei Kogyo Co., Ltd.)]. The assay method is as follows. Tris below is an acronym of tris(hydroxymethyl)aminomethane and M represents the concentration unit Mol/L.

1 ml of 200 μM DPPH solution in ethanol was added to 800 μl of 100 mM Tris-HCl buffer, pH 7.4 and 200 μl of a test compound solution in ethanol (to a final concentration of 10 μg/mL), for sufficient agitation. After the resulting mixture was left to stand alone in darkness at ambient temperature for 20 minutes, the absorbance at 517 nm was measured (absorbance of the test solutions).

Meanwhile, 800 μl of 100 mM Tris-HCl buffer, pH 7.4, 200 μl of ethanol and 1 ml of 200 μM DPPH solution in ethanol were used for the same procedures as described above to measure the absorbance to obtain the absorbance of the blank.

The individual test compounds and the blank were repeatedly treated by the same procedures three times, and the mean of the resulting absorbance values was inserted in the following formula, to calculate the ratio of scavenging DPPH radical.

Ratio of scavenging DPPH radical (%) =

$$\{1\text{-(absorbance of sample solution/absorbance of blank solution)}\} \times 100$$

The results are shown below. The compound of the formula (11) and the compound of the formula (12) had an ability of scavenging radical at higher levels than that of dl-α-tocopherol.
Ratio of scavenging DPPH radical with the compound of the formula (11): 35.1 %
Ratio of scavenging DPPH radical with the compound of the formula (12): 40.0 %
Ratio of scavenging DPPH radical with dl-α-tocopherol: 26.7 %

Example 5

Using the compound of the formula (11) obtained in Example 1, a test about the inhibition of tyrosinase activity using L-dopa as substrate was done. The same test was done about arbutin (manufactured by Tokyo Kasei Kogyo Co., Ltd.), to get control data. The test procedures are as follows. The test results are shown in Fig.1.

(1) 1.80 ml of a phosphate buffer prepared by dissolving 1.000 g of sodium dihydrogen phosphate and 1.186 g of disodium hydrogen phosphate in 500 ml of distilled water, 1.00 ml of a substrate solution prepared by dissolving 32.7 mg of L-dopa in 200 ml of distilled water, and 0.10 ml of a test solution prepared by dissolving the test compound, namely the compound of the formula (10) in dimethylsulfoxide were mixed together. The amount of the test compound used was an amount adjusted to a content of the test compound being 0.27 μM or 0.54 μM in 3 ml of the enzyme reaction solution obtained by the following procedure (2).
(2) 0.10 ml of an enzyme solution prepared by dissolving 3.0 mg (2400 units; manufactured by Sigma Corporation) of mushroom tyrosinase in 7.0 ml of distilled water was added to the solution prepared in (1), for agitation for 15 seconds. The resulting solution was retained at 25 °C, to measure the absorbance at 475 nm one minute-45 seconds later and 2 minute- 45 seconds later.
(3) The test procedures above in (1) and (2) were conducted three times for the individual test compounds and blank test (only dimethylsulfoxide), and the resulting mean was inserted in the following calculation formula to determine the ratio (%) of the inhibition of tyrosinase activity.

$$\text{Ratio of inhibition of tyrosinase activity (\%)} = [(T^1-T^2)/T^1] \times 100$$

$T^1$ = difference in absorbance of blank solution between 2 minute-45 seconds later and one minute-45 seconds later
$T^2$ = difference in absorbance of solution with addition of test compound between 2 minute-45 seconds later and one minute-45 seconds later

Fig.1 shows that the compound of the formula (11) had a property superior to that of arbutin as an existing inhibitor of tyrosinase activity.

Industrial Applicability

The gingerol analogues of the invention has an excellent inhibitory action of tyrosinase activity and a great antioxidant action and can be blended in foods, pharmaceutical products, qui-pharmaceutical products and cosmetics such as cosmetic lotion, aesthetic lotion, emulsion, cream and pack, and the like.

## Claims

**1.** Gingerol analogues represented by the following general formula (1):

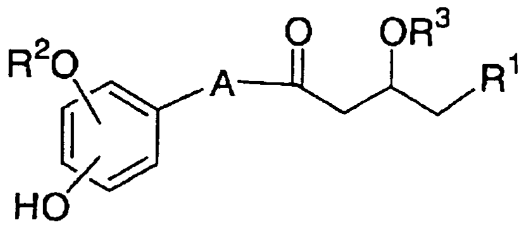

(1)

(in the formula (1), $R^1$ represents an alkyl group with one to 18 carbon atoms; $R^2$ is hydrogen atom or methyl group; $R^3$ is methyl group or ethyl group; and A represents an alkylene group with one to 4 carbon atoms.)

**2.** Gingerol analogues represented by the following general formula (2):

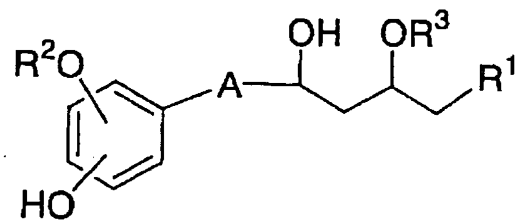

(2)

(in the formula (2), $R^1$ represents an alkyl group with one to 18 carbon atoms; $R^2$ is hydrogen atom or methyl group; $R^3$ is methyl group or ethyl group; and A represents an alkylene group with one to 4 carbon atoms.)

**3.** A tyrosinase activity inhibitor comprising gingerol analogues according to claim 1 or 2.

**4.** An antioxidant comprising gingerol analogues according to claim 1 or 2.

FIG. 1

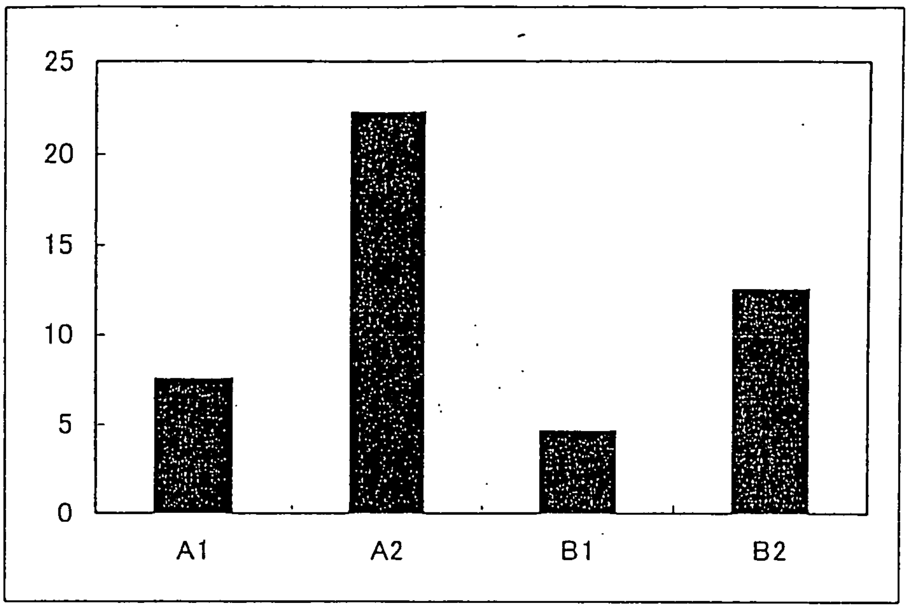

## INTERNATIONAL SEARCH REPORT

International application No.
PCT/JP03/06470

A. CLASSIFICATION OF SUBJECT MATTER
Int.Cl[7] C07C43/178, 43/23, 49/255, A61K7/00, 7/48, 31/075, 31/085, 31/12, A61P17/00, 39/06, 43/00

According to International Patent Classification (IPC) or to both national classification and IPC

B. FIELDS SEARCHED

Minimum documentation searched (classification system followed by classification symbols)
Int.Cl[7] C07C43/178, 43/23, 49/255, A61K7/00, 7/48, 31/075, 31/085, 31/12, A61P17/00, 39/06, 43/00

Documentation searched other than minimum documentation to the extent that such documents are included in the fields searched

Electronic data base consulted during the international search (name of data base and, where practicable, search terms used)
CA(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| A | Sachico SHIROTA, Kouji MIYAZAKI, Ritsuo AIYAMA, Minoru ICHIOKA, Teruo YOKOKURA, "Tyrosinase Inhibitors from Crude Drugs", Biological & Pharmaceutical Bulletin, 1994, Vol.17, No.2, pages 266 to 269 | 1-4 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 07 August, 2003 (07.08.03) | 26 August, 2003 (26.08.03) |
| Name and mailing address of the ISA/ Japanese Patent Office | Authorized officer |
| Facsimile No. | Telephone No. |

Form PCT/ISA/210 (second sheet) (July 1998)